Europäisches Patentamt

European Patent Office

Office européen des brevets

(11) Publication number: **0 417 952 B1**

## EUROPEAN PATENT SPECIFICATION

(45) Date of publication of patent specification: **07.12.94**  (51) Int. Cl.⁵: **B23K 26/06**, A61F 9/00

(21) Application number: **90309611.3**

(22) Date of filing: **03.09.90**

(54) **Shaping using area patterning mask.**

(30) Priority: **05.09.89 US 402946**

(43) Date of publication of application:
**20.03.91 Bulletin 91/12**

(45) Publication of the grant of the patent:
**07.12.94 Bulletin 94/49**

(84) Designated Contracting States:
**CH DE FR GB IT LI NL SE**

(56) References cited:
**EP-A- 0 257 836**
**EP-A- 0 265 872**
**US-A- 4 620 288**

(73) Proprietor: **COLLOPTICS, INC.**
**2500 Faber Place**
**Palo Alto, California 94303 (US)**

(72) Inventor: **Rose, James Wilson**
**482 Kenwood Avenue**
**Delmar, New York 12054 (US)**
Inventor: **Levinson, Ronnen M.**
**1 Linda Lane**
**Schenectady, New York 12309 (US)**
Inventor: **Liu, Yung Sheng**
**2201 Pine Ridge Court**
**Schenectady, New York 12309 (US)**

(74) Representative: **Goldin, Douglas Michael et al**
**J.A. KEMP & CO.**
**14, South Square**
**Gray's Inn**
**London WC1R 5LX (GB)**

# Description

The present invention relates to the field of workpiece shaping, with important application in the field of laser shaping of a workpiece using a mask to control workpiece exposure.

There is presently substantial interest in the use of ultraviolet laser ablation of the human cornea to reshape the cornea to correct vision problems such as myopia, hyperopia and astigmatism. Such laser sculpturing is accomplished by the ablation of a thin layer of the cornea upon exposure to a pulse of ultraviolet light. The preferred ultraviolet light has a wavelength of less than 200 nanometers. For pulse fluence of 200 mJ/cm$^2$, a layer of corneal material about 0.1 to 0.2 microns thick is removed with each pulse. A variety of techniques have been proposed for controlling the laser ablation in manner to provide a desired change in the shape of the corneal surface. These include the use of mask with a central aperture or slit therein configured to restrict each pulse of the laser beam to impinging on a small portion of the corneal surface so that scanning the mask around the cornea eventually removes the corneal material in the desired manner. Another technique is the use of a small diameter spot scanned across the cornea to, with many pulses, cumulatively remove the corneal material in the desired manner.

All of these techniques suffer from several drawbacks. They work on the cornea in localized areas in succession with the result that they are quite time consuming because of the large number of pulses which must be employed to complete the reshaping of a cornea. Many of these processes require several minutes or more to complete the resculpturing of a cornea. Such a time period has a number of disadvantages. The patient's head and eye must remain in a fixed position for the entire sculpturing time in order for accurate sculpturing to take place. This places a strain on the patient and the ophthalmic surgeon. The longer the process takes, the greater the costs of the procedure. Each of these techniques requires a large number of laser pulses to accomplish a desired reshaping. The use of a mask with a slit to control the exposed area, is inherently inefficient in the use of the laser energy in that a large portion of the laser energy is blocked by the mask. For the scanning spot type of ablation control, relatively enormous times are required in order to accurately remove the desired quantity of material in each small area of the cornea.

With each of these techniques, the accuracy of the reshaping of the corneal surface is directly dependent on the accuracy with which the mask and/or laser beam are scanned across the cornea and the accuracy with which the laser energy per pulse is controlled. There is a need for a laser sculpturing technique which takes only a few seconds to sculpture a cornea or lenticule and a technique whose accuracy is not dependent on accurate scanning of a laser beam or aperture in a mask across the corneal surface.

Each of these systems also suffers from a safety risk that a system failure during the ablative sculpturing of a cornea can lead to incorrect sculpturing of the cornea including removal of more material than desired. Such risks are of particular concern in the direct sculpturing of a cornea for several reasons. As is well known, the human eye, and in particular, its cornea, is not capable of rapid regeneration, if capable of regeneration at all and is subject to scarring which interferes with clear vision. Consequently, undesired removal of material is a non-reversible process which can permanently damage the patient's eyesight. Further, since each individual only gets one set of eyes and because of the great importance of visual information in our modern information-rich society, individuals are cautioned to take extreme care of their eyes.

Even the most reliable electronic systems are subject to occasional failures. In the event of failure during a sculpturing process, permanent damage to the eye could occur.

A sculpturing technique which ensures against unintentional removal of tissue is highly desirable for use in sculpturing the human cornea.

EP-A-2 57 836 discloses a method of controlled ablation of a cornea using ultraviolet laser radiation, wherein a predetermined set of masks are placed in time-sequential manner in the path of the laser beam, each mask having the same shape (rectangular, circular, annular, elliptical) but having a different aperture to restrict the width of the light beam. In this way, a predetermined sculpturing action is developed. However, the process is time-consuming involving placing a large number of masks in the light-beam in time-sequential manner, and further the structure for supporting the masks is bulky and complex.

The present invention, as defined in claim 1, provides a plurality of mask patterns which each comprise a plurality of small pixels, each of which controls passage of energy to an individual small area of the workpiece and for each of the mask patterns to control passage of a different amount of energy whereby the required shaping can be carried out. It is preferred to move at least the or those masks transmitting most energy through a small distance less than the dimension of a pixel during an energy transmission therethrough.

In some embodiments, each mask pattern is provided on a substrate individual thereto, to provide a set of binary weighted masks, while in an alternative arrangement, a plurality of said mask patterns are provided on a single substrate to provide a mask with a range of different transmissivities for different pixels.

There is disclosed herein a laser shaping technique which is not dependent on scanning of either the laser beam or an aperture in a mask and in which the entire area to be shaped may be treated as a unit, with accurate precontrol of the pattern of shaping of the workpiece.

To these ends and others which will become apparent from the specification as a whole, taken in conjunction with the drawings, the shaping laser beam is passed, in accordance with the present invention, through an area patterning mask which accurately controls the pattern in which each laser pulse shapes the workpiece.

The masks may preferably have binary weights in which a first mask of weight 1 is used to control the exposure pattern for K laser pulses, a mask of weight 2 is used to control the pattern of laser exposure for 2K pulses, a mask of weight 4 is used to control the area of exposure for 4K laser pulses, and so forth. Non-binary weights may be employed, if desired.

The invention both as to organization and method of practice, together with further objects and advantages thereof, may best be understood by reference to the following description taken in connection with the accompanying drawings in which:

Figure 1 is a table relating the relative degree of ablation desired to the transparency (1) or opaqueness (0) of the corresponding pixel in each of a set of weighted masks;

Figures 2 and 3 each illustrate four pixel patterns of desired relative ablations and the corresponding opaque or transparent state of the corresponding pixels in each of four masks;

Figure 4 illustrates eight weighted mask patterns A-H which together form a convex spherical surface on a flat workpiece;

Figure 5 is a perspective, partially cutaway view of a workpiece sculptured by the mask set of Figure 4;

Figure 6 illustrates eight weighted mask patterns A-H which together form a hyperbolic paraboloidal surface on a flat workpiece;

Figure 7 is a diagrammatic illustration of the hyperbolic paraboloidal surface produced by the mask set of Figure 6;

Figure 8 graphically illustrates the resolution of laser ablation as a function of the number of weighted masks and pixel size;

Figure 9 is a cross-section along a diameter of a section of a spherical surface having a radius of curvature $R_1$;

Figure 10 is a cross-section along a diameter of a section of a second spherical surface having a radius of curvature $R_2$, $R_2 > R_1$;

Figure 11 is a cross-section along a diameter of the spherical surface of Figure 9 illustrating the material to be removed to convert the spherical surface of Figure 9 into the spherical surface of Figure 10;

Figure 12 is a schematic diagram illustrating the depth of material to be removed along the cross-sectional diameter of Figure 11 as a function of position;

Figure 13 is an illustration of an eight mask set for accomplishing the ablative removal of the designated material in Figure 11 or 12;

Figure 14 is an illustration of an eight mask set for reducing the radius of curvature for a spherical surface; and

Figure 15 illustrates conversion of one arbitrary surface to another arbitrary surface.

In accordance with the present invention, rather than sculpturing a workpiece such as the human cornea (or a lenticule for attachment to the human cornea) or other workpiece by scanning a sculpturing laser beam across the workpiece, an area mask is provided for controlling the transmission of laser energy to the entire working area of the workpiece for each laser pulse, with the mask controlling which areas of the workpiece are subject to shaping or ablation by each pulse. This has the advantage, that the pattern of ablation is controlled by the mask, rather than by scanning the laser beam or by scanning an aperture to control the scanning of the laser beam. In this manner, the pattern of ablation is controlled by the mask which is prefabricated and whose pattern can be checked prior to its use for laser shaping to ensure that the pattern actually present on the mask will produce the desired pattern of shaping.

In accordance with one preferred embodiment of a mask in accordance with this invention, a pattern of ablation is controlled by a set of weighted masks in which the degree of material removed in alignment with a transmissive portion of a mask is proportional to the weight assigned that mask. A particularly preferred embodiment of a weighted mask set comprises a set of binary weighted masks in which successive masks have weights of 1, 2, 4, 8, 16, 32, ... with the number of masks and the maximum weight being dependent on the desired resolution in the removal of material.

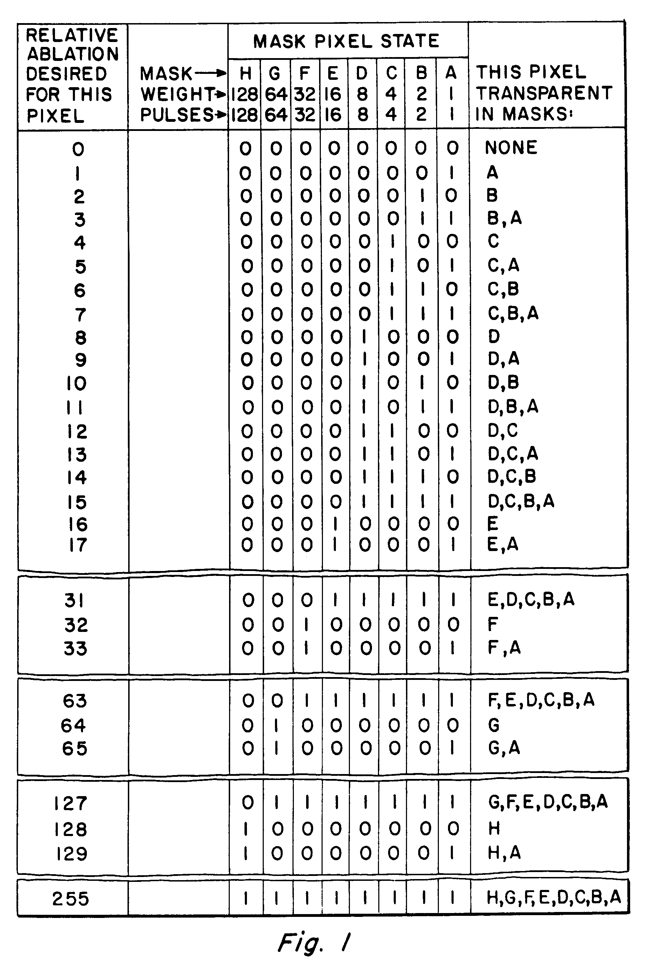

Referring now to Figure 1 which is a table relating the degree of ablation desired for a given pixel to the mask state for that pixel in each mask of a weighted set. The relative degree of ablation desired increases from 0 to 255 ($2^7$ - 1) in the lefthand column of this table with gaps after 17 in the table. In the middle column of this table are a series of binary numbers, in the top row of the heading above these numbers are mask letters which start with the lowest weight mask A at the right and increase in alphabetical order to the highest weight mask H at the left. Directly under these mask letters are the corresponding weights for the masks which increase in powers of 2 from 1 on the right to 128 on the left. Directly thereunder, are the number of laser pulses to be transmitted through each mask for equal energy laser pulses which number increase from 1 on the right to 128 on the left. In the righthand column of the table is a list by mask letter of the masks in which the corresponding pixel is transparent for the specified relative degree of ablation on that same row of the table. The upper part of the table (where the relative degree of ablation desired increases by 1 from row to row) only encompasses five different masks A-E. Lower down in the table, relative degrees of ablation are shown where an additional mask is needed, with gaps in order to fit the table on a single sheet. Those skilled in the art will recognize that the omitted relative degrees of ablation are controlled by the pixel states of the mask in a manner similar to that in which the earlier table values (lower relative degrees of ablation) are controlled.

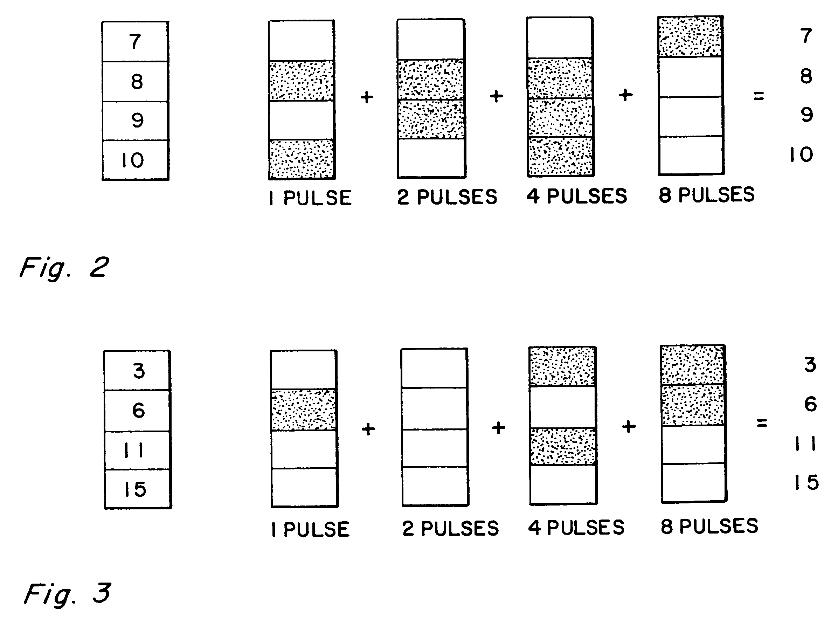

In Figure 2, a set of four pixels with relative degrees of ablation of 7, 8, 9 and 10 is illustrated at the lefthand side of the figure. The corresponding states for these four pixels in masks A, B, C and D having weights of 1, 2, 4 and 8, respectively, are illustrated across the middle portion of the figure. The total number of pulses striking the associated portion of the workpiece is indicated at the far right of the figure. In Figure 2, a filled-in pixel in the mask in the middle portion of the figure is opaque to the laser pulse, while an empty pixel is transparent to the laser pulse. The number of equal energy pulses transmitted through each mask is indicated directly below that mask in the middle portion of the figure.

Figure 3 is similar in configuration to Figure 2, but illustrates a situation with four different pixels with relative degrees of ablation of 3, 6, 11 and 15.

It will be understood that where the degree of shaping is proportional to energy density of the laser pulse, the energy per pulse could be doubled for each increase in mask weight rather than doubling the number of pulses of constant energy. That is, where shaping is linear with cumulative energy, it is the cumulative energy controlled by the mask which is important. Where material shaping is non-linear with pulse energy, appropriate constant energy pulses are preferred with their number increasing with mask weight. However, appropriate combinations of pulse energy and the number of pulses may be selected and used if desired.

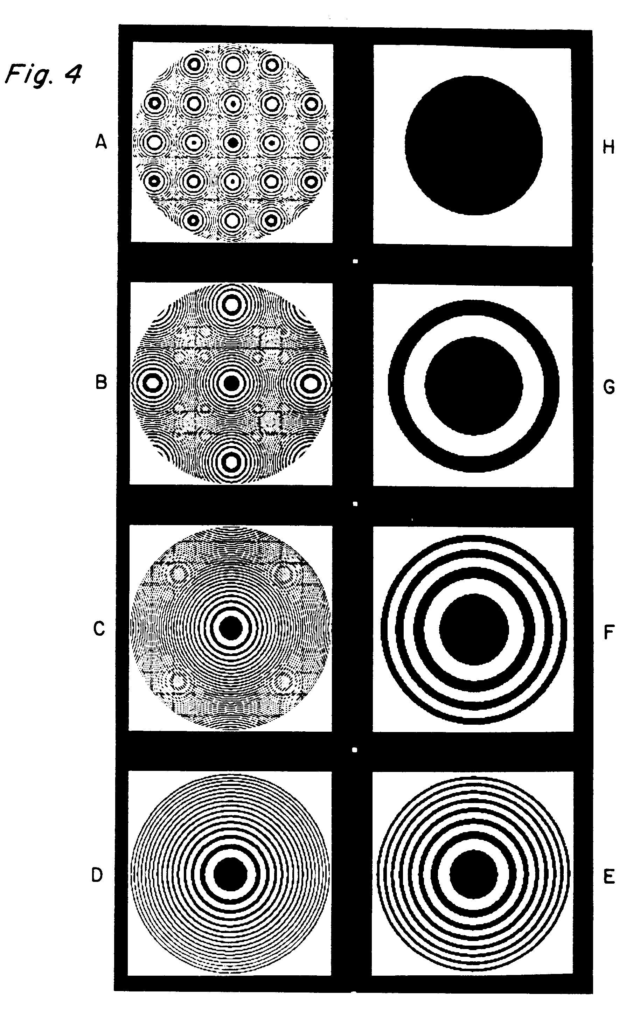

Figure 4 illustrates eight binarally weighted masks which when used in weighted combination, remove material from a flat workpiece in a configuration to leave a portion of a convex, spherical surface on the exposed face of the workpiece. The mask A has the least weight and has a single pulse transmitted therethrough. The mask B has a weight of 2 and has 2 pulses transmitted therethrough. The mask C has a weight of 4 and has 4 pulses transmitted therethrough. In a similar fashion, the mask D has a weight of 8, the mask E has a weight of 16, the mask F has a weight of 32, the mask G has a weight of 64 and the mask H has a weight of 128. It will be observed, that the geometric center of each of these mask patterns is opaque with the result that no material is removed in the center of the workpiece, while beyond the outer limit of the detailed patterning in pattern A all masks are transparent whereby a quantity of material which is equal to the sum of the weights of all the masks times the thickness of material removed by each pulse, is removed by laser ablation controlled by this mask set. In this case, a total depth of 255 times the depth removed by 1 pulse is removed in this peripheral portion of the workpiece.

If it were desired to remove more material in the periphery of the workpiece outside the region of the dome whose surface is a portion of a sphere so as to make the dome the top of a cylinder extending above the background portion of the workpiece, this could be done by adding a mask "I" which is opaque in the area of detailed patterning in pattern A and transparent outside that circular area and exposing this mask with a weight corresponding to the desired depth of removal. Alternatively, twice the material could be removed at the periphery of the workpiece with a decrease in the smoothness of the material surface simply by doubling the number of pulses transmitted through each of the 8 masks thereby doubling the amount of material removed at each location within the work zone. However, this would result in a surface which is a portion of a non-spherical elipsoid rather than a portion of a spherical elipsoid.

Each of the masks A-H is preferably disposed on a single mask substrate with each of the mask patterns having its center in a known location relative to a reference location which may be within one of the masks or mask patterns (such as at its center) or may be outside all of the patterns.

One characteristic which will be noted in the mask set of Figure 4 for sculpturing a flat workpiece to have a spherical surface is that the detail present in the masks decreases with increasing mask weight. This characteristic is common to many mask sets designed to remove a volume having a smooth symmetric boundary.

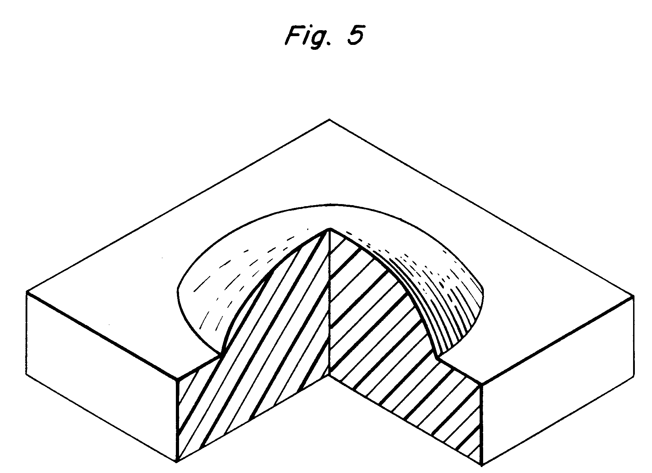

Figure 5 is a perspective, partially cutaway view of a workpiece sculptured using the mask set of Figure 4 to convert a flat upper surface into one having a surface including a spherical contour.

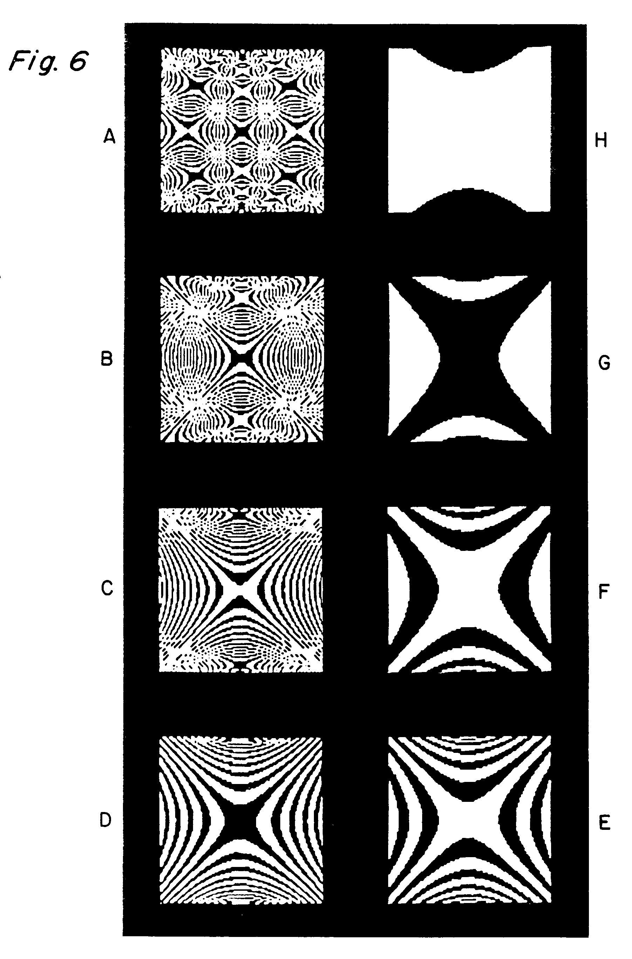

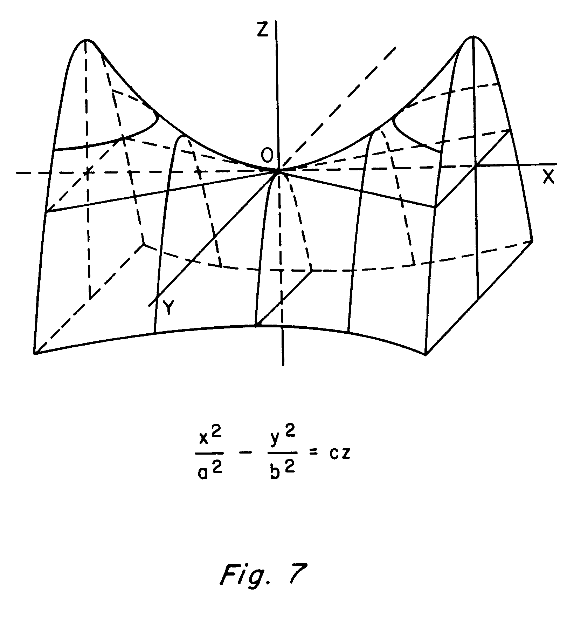

In Figure 6, an eight mask set for forming a hyperbolic paraboloidal surface is shown. In Figure 7, the resulting surface is shown, but rotated 90° relative to the mask set in that in Figure 7, the lefthand and righthand sides of the surface are formed by the upper and lower portions of the mask patterns in Figure 6.

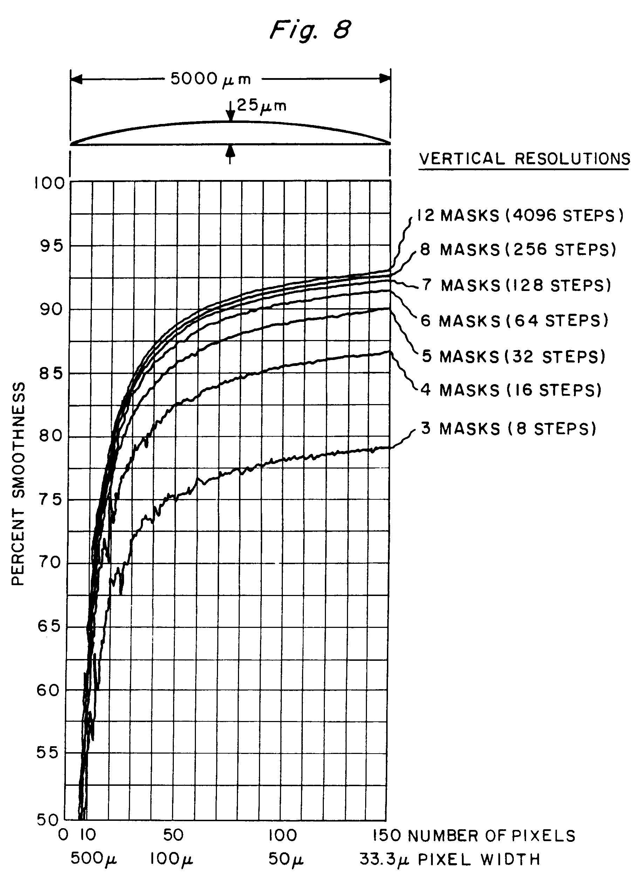

One thing which is of interest for the shaping of optical surfaces such as the surface of a lenticule or a human or animal cornea is the degree of smoothness of the final surface produced by laser ablation using a binary mask set in accordance with the present invention. While a wide variety of criteria can be used to measure that smoothness, we have chosen one standard for use in the comparison of the resolution obtained by a given number of masks and as a function of the pixel size. Where a flat workpiece is sculpted to produce a convex spherical surface, the standard we have chosen is the ratio of the volume of the material actually removed by the laser ablative process to the volume of material which would have been removed to create a spherical surface which is the largest spherical surface which is located entirely within the workpiece or at its surface. This standard is illustrated in graphical form in Figure 8, where the sculptured area is 5,000 microns in diameter and is sculptured to have a surface which is a portion of a sphere and 25 microns higher at the center of the area than at its edge, as shown in cross-section at the top of Figure 8. A smoothness percentage of 100 would characterize a surface which was everywhere coincident with a perfect spherical surface. As can be seen, the smoothness percentage is low for a small number of pixels independent of the number of masks used. This is because a small number of pixels results in large, flat topped areas which are spaced in major portion from the "ideal" spherical surface, independent of the fineness of the vertical (depth) control. It will be noted, that the smoothness increases with the number of masks in the mask set (and thus, the number of different depths of removal available and thus the fineness of the vertical resolution) and with increases in the number of pixels (and thus the fineness of the horizontal resolution). It will also be recognized that the thinner the layer of material which is removed by each laser pulse, the better the smoothness of the resulting curve will be, although the maximum quantity of material which can be removed by a given number of pulses will decrease.

Figure 8 illustrates the smoothness for one particular sculpturing situation. Other situations will have other smoothnesses. Where the workpiece is an optical element, the degree of smoothness needed will depend on the particular optical element and the overall system in which is is used.

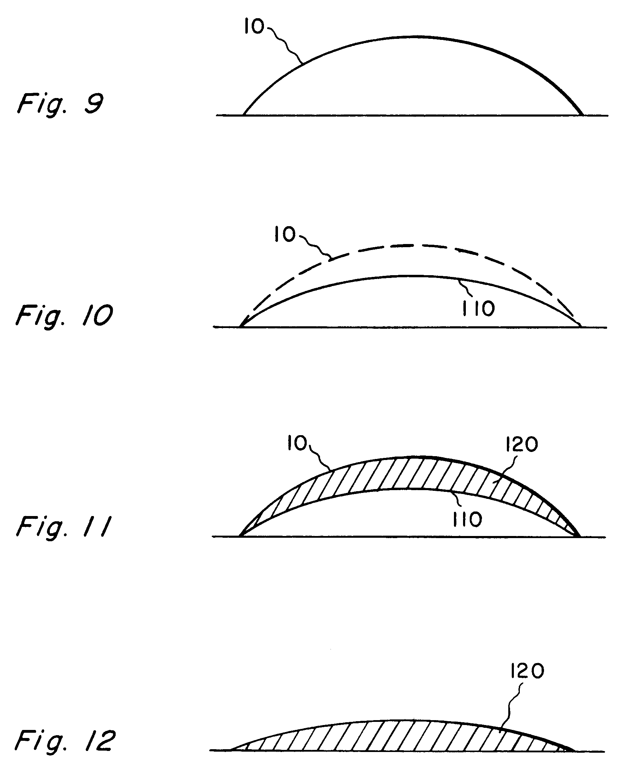

In general, for sculpturing the human cornea or a lenticule attached to a cornea, the problem faced is not one of machining a flat workpiece to produce a spherical surface, but rather one of sculpturing an existing curved surface to convert it to a different curved surface which results in improved vision. In Figure 9, an initial curved surface 10 which it is desired to reconfigure, is illustrated in a cross-section taken along a diameter of that spherical surface. In Figure 10, a desired final curved surface 110 is illustrated along with, in dotted outline, the existing initial surface 10.

In Figure 11, the initial curved surface 10 is illustrated with the desired curved surface 110 disposed inside thereof (since only material removal is presently feasible with this process) with the material 120 to be removed cross-hatched for clarity.

In Figure 12, the material 120 to be removed is illustrated as a thickness to be removed as a function of position along the diameter of the surface 10. It will be noted, that this thickness is 0 at the edges of the surface and increases monotonically to the center of the surface.

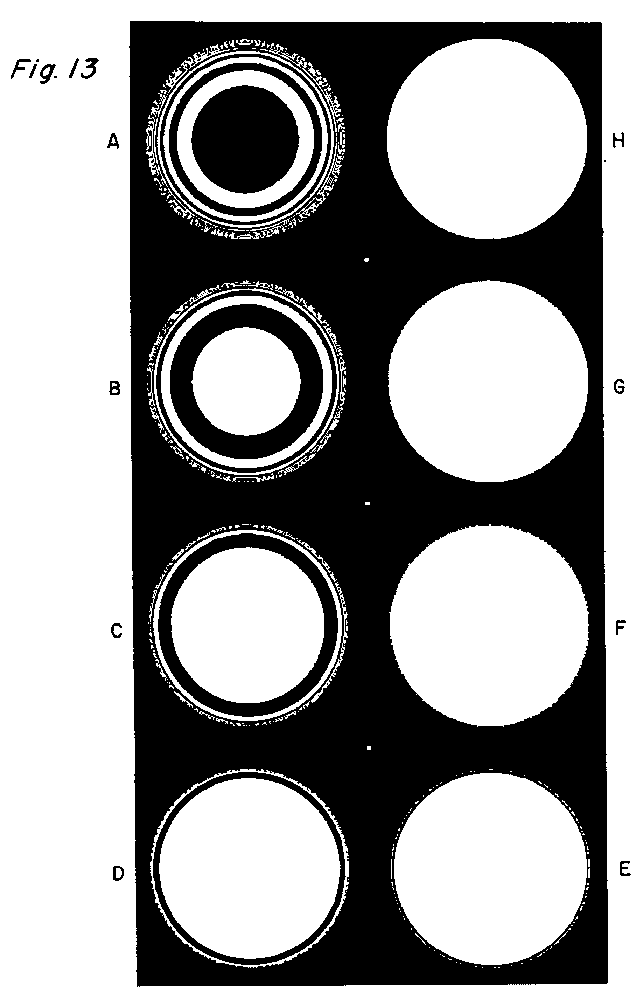

In Figure 13, an 8 mask set for removing the material 120 is illustrated.

Few human corneas are perfectly spherical. Also, a perfectly spherical corneal surface will provide optimum vision for very few people. Human corneas have irregular shapes and the overall human optical system is such that typically, an irregular shape will provide an individual with optimum vision. Consequently, the optimum ophthalmic use of this invention is in converting one irregular curved surface to a different irregular curved surface.

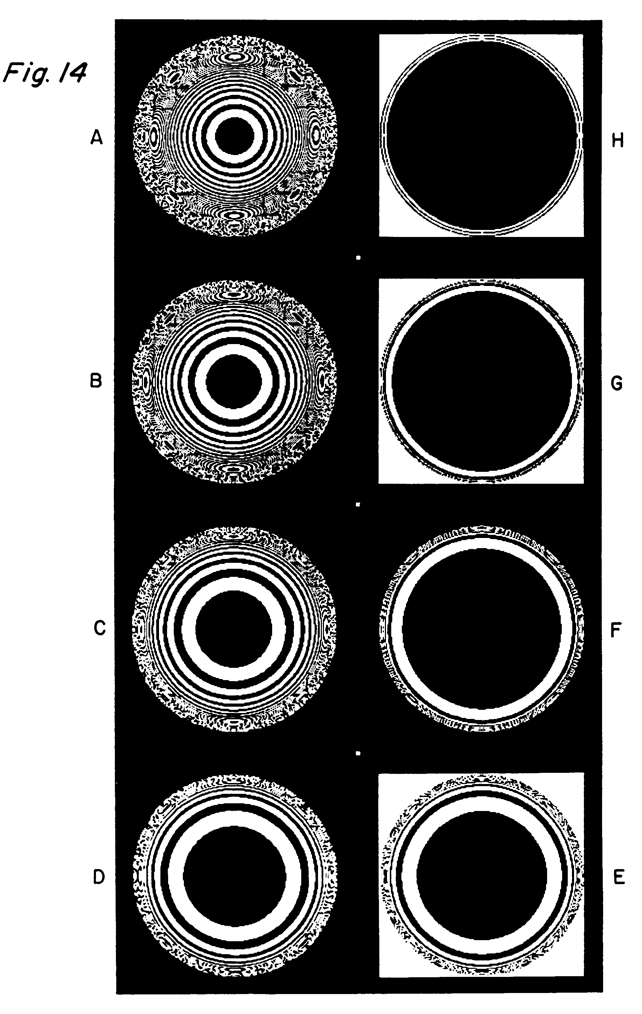

Figure 14 is an eight mask set for reducing the radius of curvature (increasing the curvature of) a spherical surface. This mask set changes curvature in the opposite way from that shown in Figures 10-13.

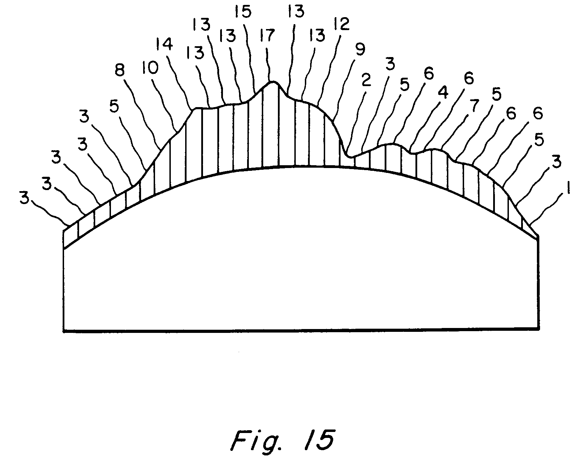

One of the advantages of the binary weighted area pattern mask embodiment of this invention is that in each pixel a quantity of material ranging from zero to $(2^N-1)$ times the depth of material removed by one pulse can be removed. Thus, any arbitrary surface can be converted to any other arbitrary included surface by defining the quantity of material to be removed in each pixel. Figure 15 provides a coarse example of this along a cross-section through a body of material. The number above each pixel corresponds to a thickness to be removed in that pixel.

This invention provides a substantial number of advantages over prior art techniques for sculpturing corneas, lenticules and other workpieces and for the conversion of one irregular surface to a different irregular surface. In particular, the degree of material removal is controlled during the mask making process with the result that detailed beam movement and material removal profiles of an irregular nature do not have to be produced by a detailed and carefully controlled movement of a spot laser beam or an aperture mask. Consequently, more reliable control of the actual material removal profile is provided by the present invention as compared to the prior art processes. Further, since the entire sculpturing process is performed by a laser beam which is large enough to encompass the entire patterned mask area, many fewer pulses are required to produce a desired profile. In particular, where a maximum removal depth of 255 times the depth removed by a single pulse is desired, only 255 laser pulses are required for the entire sculpturing process, rather than 255 laser pulses being required by the small, scanned spot process at each point at which removal of that depth of material is desired. Consequently, this mask set drastically reduces the number of laser pulses required to sculpture a cornea, lenticule or other workpiece. This provides many advantages. First, the process is much faster than prior art processes. Second, the number of laser pulses to which the eye is exposed is drastically reduced, thereby minimizing any risks of unexpected side effects from that laser exposure. Where an excimer laser having a pulse rate of up to 100 pulses per second is employed for this laser sculpturing process with a resolution of 1 in 255, only 255 pulses are required which corresponds to slightly more than 21/2 seconds of laser time. Since eight different masks are required for that degree of resolution, the time to switch eight different masks into proper alignment with respect to the cornea or lenticule must be added to this actual laser time. However, with modern positioning and alignment equipment, such mask switching and alignment should be accomplished in under a second for each mask switch with the result that the entire sculpturing process can be accomplished is roughly 10 seconds. Mask switching times of much less than one-half second are possible with appropriate equipment. Thus, the entire process can be conducted in well under 10 seconds. This greatly simplifies the problem of the patient holding his or her eye still during the laser ablation process, since it is much easier for a patient to hold an eye fixed at a certain location for 10 seconds than for several minutes. Further, the correct pattern of ablation is assured for correctly fabricated masks which are properly aligned so long as the requisite number of constant energy pulses are transmitted through each mask pattern.

In order to minimize the risk of operator error or other interference with the proper completion of the laser ablation process in accordance with this invention, it is preferred to have all of the mask patterns for a given laser ablation sculpturing defined in a masking layer on a single substrate having an alignment key which provides positive assurance that the mask is properly asserted in the sculpturing equipment. This alignment key may be part of the physical configuration of the substrate, part of the pattern on the substrate as shown in the lower right in Figure 6, or both. A similar cut on the corner of the substrate may serve as an alignment key. Other alignment keys may be used. Each such mask substrate should also include positive identification of the patient and eye for which is intended. This may be in the form of words such as the patient's name and "left eye" or "right eye" or may be numbers or other symbols. It is preferred to have the sculpturing equipment include automatic interlocks which limit the number of pulses transmitted through each mask set in accordance with its weight. It is also desirable to have positive alignment marks on the masks which ensure that the masks are properly aligned with the lenticule or cornea.

One technique for providing such positive alignment is to sculpt four alignment marks outside the visually active portion of the cornea or lenticule with which alignment marks in each of the masks are aligned prior to transmission of the ablative laser pulse through the mask. Such alignment references may be sculptured in the cornea or lenticule or may be small reflective spots added to the cornea or lenticule or points illuminated by position reference beams which are viewed through the mask by an alignment system.

While this invention has been described in terms of binarally weighted masks, other mask weights may be used with equal generality. The primary advantage of binarally weighted masks is that a minimum number of masks for a given resolution of material removal results.

Masks with pixels which are from 3 to 6 microns square can be fabricated using a scanned laser beam to expose a photoresist pattern or to directly ablate a masking layer from a mask substrate.

As an alternative mask preparation technique, the mask pattern can be printed with a laser printer at 300 dots per inch (1 inch = 25,4 mm), and reduced by four to one to provide a pattern at 1,200 dots per inch or 20.8 micron wide pixels.

These masks may be used as near contact masks with parallel beam laser light as "contact" masks providing 1:1 mask to workpiece pattern sizing. Enough space must be left between the mask and the workpiece for removal of the ablation products. Alternatively, these masks may be used as projection masks with size reduction between the mask and the workpiece. With projection masks, it is preferred that the laser light be parallel rays at the workpiece surface to avoid distortion due to the workpiece work surface being non-planar on a microscopic scale.

For some laser shaping work with small enough pixels, laser beam fringing at the edges of transparent areas of the mask may affect the material removal profile. Such effects can be minimized for high weight masks by shifting them slightly after each laser pulse or after a prescribed number of laser pulses. This "slight" movement should be less than a pixel width and preferably less than one-half of a pixel width.

In general, fabrication of the masks is simplest if the mask substrate has a planar surface. However, it may be desirable to form the mask on a shaped surface to control fringing effects or for other purposes. Such shaped surfaces include those having a cornea-like curvature which places the masking layer at a roughly constant distance from the corneal surface.

The masking layer may be disposed on the surface of the transparent mask substrate which is toward the workpiece or away from the workpiece.

In order to simplify the process of fabricating masks of this type, it is preferred to have automatic mechanisms for storing mask blanks, loading mask blanks into an exposure station and defining the mask patterns thereon. The process of defining the mask patterns may preferably comprise exposing and developing a photoresist layer of the mask blank and then removing an opaque coating from the surface of the mask substrate in a pattern determined by the photoresist. Equipment of the general type used for handling and processing semiconductor wafers can be used for this purpose. An adaption of a laser printer may be used as the exposure mechanism.

The masks described herein have assumed that the mask pattern is binary in that each pixel is either 100% transmissive or 0% transmissive. Naturally, a mask substrate which is 99% transmissive may be used, without significant deviation from the desired sculpturing pattern. While more difficult to fabricate, a mask pattern consisting of 100% transmissive portions, 50% transmissive portions and 0% transmissive portions may be fabricated and used in accordance with the present invention by providing a mask blank having an initial mask layer thereon which is 50% transmissive and a second mask layer thereover which is 0% transmissive. Such masks may be patterned by first defining a photoresist pattern which results in the removal of the 0% transmissive layer everywhere except where 0% transmission is desired. Thereafter, a second photoresist layer is patterned to remove the 50% transmissive layer wherever 100% transmission is desired. In this manner, a multilevel mask is provided which can reduce the number of masks required. In a similar manner, if desired, a mask having 0%, 25%, 50%, 75% and 100% transmissive regions could be provided. The ideal mask of this type for producing an arbitrary and smooth sculptured surface would range in transmission between 0 and 100% in accordance with the desired degree of ablation of the underlying material. Such a mask is within the concept of the present invention in that it is an area mask which defines the pattern of ablation over the entire sculpturing area.

One method of producing such an analog area mask is to use a holographic pattern to convert the uniform incident laser pulse into a pattern of light which varies in intensity with position in accordance with the desired degree of ablation. Such a holographic mask may be used as a single mask to control ablation or weighted holographic masks may be employed where finer resolution is desired.

The laser beam should encompass an entire mask pattern and have a fluence which is constant across the mask pattern for proper weighted removal of material. However, if the beam has a known variation in fluence across the beam, this can be compensated for during the mask design process by determining for each pixel the depth to which it is desired to remove material, the total fluence required for that removal and the fluence of the laser beam at the location of that pixel. The total fluence required is then divided by the local beam fluence to determine the number of pulses needed. That number of pulses is then built into the mask by the state of that pixel in each of the patterns of the mask.

Much of the discussion of this invention has been in terms of its use for sculpturing lenticules and corneas. However, this invention can be used for the shaping of any laser shapeable workpiece, including plastics, metallic bodies, and so forth.

For the purposes of the claims, the term workpiece does not include a cornea.

The maximum depth (D) of material to be removed, the number (n) of pulses required and the thickness (T) removed by one pulse are related as:

$$D/N = T$$

Thus,

$$D = NT$$
$$T = D/N \text{ and}$$
$$N = D/T$$

While the invention has been described in detail herein in accord with certain preferred embodiment thereof, many modifications and changes therein may be effected by those skilled in the art.

For instance, while the foregoing description has concentrated on laser shaping, the invention may at least in some cases be practised using forms of beam energy other than laser light.

## Claims

1. A method of sculpturing a workpiece (10), comprising:

   providing a set of mask patterns (A-H), each comprising an individual pattern of a large number of transmissive pixels, each mask pattern encompassing the entire working area of the workpiece, said transmissive pixels corresponding to locations in which it is desired to remove a quantity of material proportional to the weight of that mask pattern;

   providing a source of ablative energy which provides a beam of sufficient size to encompass each of said mask patterns individually;

   positioning said mask patterns in the path from said source to said workpiece, and

   directing an amount of said ablative energy through each said mask pattern onto said workpiece which is effective to remove a quantity of material from the exposed portions of said workpiece which is proportional to the weight of said pattern, said ablative energy being provided as pulses of substantially predetermined energy.

2. A method according to claim 1, wherein for at least one mask pattern through which more than one pulse is transmitted, said mask pattern is shifted by less than a pixel width after at least one of said pulses directed through that mask pattern.

3. A method according to claim 2, wherein said at least one mask pattern is shifted after each pulse directed therethrough.

4. A method according to claim 1, 2 or 3, wherein said set of mask pattern has binary weights in which a first mask pattern has a weight of 1, a second mask pattern has a weight of 2 and a third mask pattern has a weight of 4.

5. A method according to claim 4, wherein said mask pattern of weight 1 is exposed with K pulses, said mask of weight 2 is exposed with 2K pulses and said mask of weight 4 is exposed with 4K pulses, wherein K is a constant integer.

6. A method according to claim 5, wherein the constant K is equal to 1.

7. A method according to any preceding claim, wherein each mask pattern is provided on a substrate individual thereto to provide a set of binary weighted masks.

8. A method according to claim 1 or 2, wherein a plurality of said mask patterns are provided on a single substrate in the form of a mask with a range of different transmissivities for different pixels.

9. A method according to claim 8, wherein the mask has 0%, 25%, 50%, 75% and 100% transmissive regions.

10. A method according to claim 8, wherein the range of transmissive regions are such as to provide an analogue area mask.

11. A method as claimed in any preceding claim, wherein said beam is a laser light beam.

12. A method as claimed in any preceding claim, wherein the workpiece is a lenticule for attachment to the cornea of an eye.

## Patentansprüche

1. Verfahren zur Formgebung eines Werkstückes (10), umfassend die folgenden Schritte:
   Bereitstellen eines Satzes von Maskenmustern (A-H), von denen jeder ein individuelles Muster aus einer großen Anzahl von durchlässigen Bildelementen umfaßt, wobei jedes Maskenmuster den gesamten Arbeitsbereich des Werkstückes umfaßt, und die durchlässigen Bildelemente Stellen entsprechen, an denen eine zum Gewicht dieses Maskenmusters proportionale Menge an Material abgetragen werden soll;
   Bereitstellen einer Quelle für Ablationsenergie, die einen Strahl liefert, der so groß ist, daß er jedes der Maskenmuster einzeln umfaßt;
   Positionieren der Maskenmuster in dem Weg von der Energiequelle zu dem Werkstück, und Richten der Ablationsenergie durch jedes Maskenmuster auf das Werkstück in einer Menge, die wirksam ist, um eine zum Gewicht des Musters proportionale Materialmenge von den freiliegenden Abschnitten des Werkstückes abzutragen, wobei die Ablationsenergie in Form von Impulsen einer im wesentlichen vorbestimmten Energie bereitgestellt wird.

2. Verfahren nach Anspruch 1, bei dem für mindestens ein Maskenmuster, durch das mehr als ein Impuls hindurchgelassen wird, das Maskenmuster um weniger als eine Bildelementbreite verschoben wird, nachdem mindestens einer der Impulse durch das Maskenmuster geleitet wurde.

3. Verfahren nach Anspruch 2, bei dem mindestens ein Maskenmuster nach jedem hindurchgeleiteten Impuls verschoben wird.

4. Verfahren nach Anspruch 1, 2 oder 3, bei dem der Satz von Maskenmustern binäre Gewichte besitzt, so daß ein erstes Maskenmuster ein Gewicht 1, ein zweites Maskenmuster ein Gewicht 2 und ein drittes Maskenmuster ein Gewicht 4 besitzt.

5. Verfahren nach Anspruch 4, bei dem das Maskenmuster mit dem Gewicht 1 mit K Impulsen belichtet wird, die Maske mit dem Gewicht 2 mit 2K Impulsen belichtet wird, und die Maske mit dem Gewicht 4 mit 4K Impulsen belichtet wird, wobei K eine ganzzahlige Konstante ist.

6. Verfahren nach Anspruch 5, bei dem die Konstante K gleich 1 ist.

7. Verfahren nach einem der vorhergehenden Ansprüche, bei dem jedes Maskenmuster auf einem dafür individuellen Substrat ausgebildet wird, um einen Satz Masken mit binärem Gewicht bereitzustellen.

8. Verfahren nach Anspruch 1 oder 2, bei dem eine Vielzahl der Maskenmuster auf einem einzigen Substrat in Form einer Maske mit einem Bereich unterschiedlicher Durchlässigkeiten für verschiedene Bildelemente ausgebildet wird.

9. Verfahren nach Anspruch 8, bei dem die Maske Bereiche mit einer Durchlässigkeit von 0%, 25%, 50%, 75% und 100% besitzt.

10. Verfahren nach Anspruch 8, bei dem die verschiedenen durchlässigen Bereich so angeordnet sind, daß eine Maske mit analogen Flächen entsteht.

11. Verfahren nach einem der vorhergehenden Ansprüche, bei dem der Strahl ein Laserlichtstrahl ist.

12. Verfahren nach einem der vorhergehenden Ansprüche, bei dem das Werkstück eine Linse ist, die auf die Hornhaut eines Auges aufzubringen ist.

## Revendications

1. Procédé de sculpture d'une pièce d'ouvrage (10), comportant :

l'apport d'un ensemble de configurations de masque (A-H), comportant chacun une configuration individuelle d'un grand nombre d'éléments d'image de transmission, chaque configuration de masque entourant la totalité de la surface d'usinage de la pièce d'ouvrage, lesdits éléments d'image de transmission correspondant à des emplacements dans lesquels on souhaite retirer une quantité de matériau proportionnelle au poids de cette configuration de masque;

l'apport d'une source d'énergie ablative, qui produit un faisceau de dimension suffisante pour entourer chacune desdites configurations de masque individuellement;

le positionnement desdites configurations de masque dans le trajet allant de ladite source à ladite pièce d'ouvrage, et

l'application d'une quantité de ladite énergie ablative à travers chacune desdites configurations de masque sur ladite pièce d'ouvrage qui est efficace pour éliminer une quantité de matériau des parties exposées de ladite pièce d'ouvrage qui est proportionnelle au poids de ladite configuration, ladite énergie ablative étant délivrée sous la forme d'impulsions d'énergie sensiblement prédéterminée.

2. Procédé selon la revendication 1, dans lequel pour au moins une configuration de masque à travers laquelle plus d'une impulsion est transmise, ladite configuration de masque est décalée de moins d'une largeur d'élément d'image après au moins une desdites impulsions dirigées à travers cette configuration de masque.

3. Procédé selon la revendication 2, dans lequel ladite au moins une configuration de masque est décalée après chaque impulsion dirigée à travers celle-ci.

4. Procédé selon la revendication 1, 2 ou 3, dans lequel ledit ensemble de configurations de masque possède des poids binaires dans lesquels une première configuration de masque possède un poids de 1, une seconde configuration de masque possède un poids de 2 et une troisième configuration de masque possède un poids de 4.

5. Procédé selon la revendication 4, dans lequel ladite configuration de masque de poids 1 est soumise à K impulsions, ladite configuration de masque de poids 2 est soumise à 2K impulsions et ladite configuration de masque de poids 4 est soumise à 4K impulsions, dans lequel K est un entier constant.

**6.** Procédé selon la revendication 5, dans lequel la constante K est égale à 1.

**7.** Procédé selon l'une quelconque des revendications précédentes, dans lequel chaque configuration de masque est prévue sur un substrat particulier à celle-ci pour constituer un ensemble de masques pondérés binairement.

**8.** Procédé selon la revendication 1 ou 2, dans lequel une pluralité desdites configurations de masque sont prévues sur un unique substrat sous la forme d'un masque avec une plage de transmissions différente pour différents éléments d'image.

**9.** Procédé selon la revendication 8, dans lequel le masque possède des régions de transmission de 0%, 25%, 50%, 75% et 100%.

**10.** Procédé selon la revendication 8, dans lequel la plage de régions de transmission est prévue de manière a constituer un masque de surface analogue.

**11.** Procédé selon l'une quelconque des revendications précédentes, dans lequel ledit faisceau est un faisceau de lumière laser.

**12.** Procédé selon l'une quelconque des revendications précédentes, dans lequel la pièce d'ouvrage est une lentille en vue d'une fixation à la cornée d'un oeil.

| RELATIVE ABLATION DESIRED FOR THIS PIXEL | MASK→ WEIGHT→ PULSES→ | MASK PIXEL STATE | | | | | | | | THIS PIXEL TRANSPARENT IN MASKS |
|---|---|---|---|---|---|---|---|---|---|---|
| | | H 128 128 | G 64 64 | F 32 32 | E 16 16 | D 8 8 | C 4 4 | B 2 2 | A 1 1 | |
| 0 | | O | O | O | O | O | O | O | O | NONE |
| 1 | | O | O | O | O | O | O | O | I | A |
| 2 | | O | O | O | O | O | O | I | O | B |
| 3 | | O | O | O | O | O | O | I | I | B,A |
| 4 | | O | O | O | O | O | I | O | O | C |
| 5 | | O | O | O | O | O | I | O | I | C,A |
| 6 | | O | O | O | O | O | I | I | O | C,B |
| 7 | | O | O | O | O | O | I | I | I | C,B,A |
| 8 | | O | O | O | O | I | O | O | O | D |
| 9 | | O | O | O | O | I | O | O | I | D,A |
| 10 | | O | O | O | O | I | O | I | O | D,B |
| 11 | | O | O | O | O | I | O | I | I | D,B,A |
| 12 | | O | O | O | O | I | I | O | O | D,C |
| 13 | | O | O | O | O | I | I | O | I | D,C,A |
| 14 | | O | O | O | O | I | I | I | O | D,C,B |
| 15 | | O | O | O | O | I | I | I | I | D,C,B,A |
| 16 | | O | O | O | I | O | O | O | O | E |
| 17 | | O | O | O | I | O | O | O | I | E,A |
| 31 | | O | O | O | I | I | I | I | I | E,D,C,B,A |
| 32 | | O | O | I | O | O | O | O | O | F |
| 33 | | O | O | I | O | O | O | O | I | F,A |
| 63 | | O | O | I | I | I | I | I | I | F,E,D,C,B,A |
| 64 | | O | I | O | O | O | O | O | O | G |
| 65 | | O | I | O | O | O | O | O | I | G,A |
| 127 | | O | I | I | I | I | I | I | I | G,F,E,D,C,B,A |
| 128 | | I | O | O | O | O | O | O | O | H |
| 129 | | I | O | O | O | O | O | O | I | H,A |
| 255 | | I | I | I | I | I | I | I | I | H,G,F,E,D,C,B,A |

*Fig. 1*

| 7 |
|---|
| 8 |
| 9 |
| 10 |

1 PULSE + 2 PULSES + 4 PULSES + 8 PULSES = 7 8 9 10

*Fig. 2*

| 3 |
|---|
| 6 |
| 11 |
| 15 |

1 PULSE + 2 PULSES + 4 PULSES + 8 PULSES = 3 6 11 15

*Fig. 3*

EP 0 417 952 B1

Fig. 4

Fig. 5

Fig. 6

$$\frac{x^2}{a^2} - \frac{y^2}{b^2} = cz$$

Fig. 7

## Fig. 8

VERTICAL RESOLUTIONS

12 MASKS (4096 STEPS)

8 MASKS (256 STEPS)

7 MASKS (128 STEPS)

6 MASKS (64 STEPS)

5 MASKS (32 STEPS)

4 MASKS (16 STEPS)

3 MASKS (8 STEPS)

Fig. 9

Fig. 10

Fig. 11

Fig. 12

Fig. 13

Fig. 14

*Fig. 15*